# EUROPÄISCHE PATENTANMELDUNG

(11) **EP 2 944 625 A1**
(43) Veröffentlichungstag der Anmeldung: **18.11.2015**
(21) Anmeldenummer: 14168561.0
(22) Anmeldetag: 16.05.2014
(51) Int. Cl.: C07C 67/03, C07C 69/54

(54) **Verfahren zur Herstellung von (Meth)-Acrylsäureestern**

(71) Anmelder: BASF SE, 67056 Ludwigshafen (DE)
(72) Erfinder: Haaf-Kleinhubbert, Christina, 69502 Hemsbach (DE); Häberle, Karl, 67346 Speyer (DE); Stein, Patrick Simon, 67067 Ludwigshafen (DE)
(74) Vertreter: Reinhardt, Jürgen

(57) **Zusammenfassung**

Die Erfindung betrifft ein verbessertes Verfahren zur Herstellung von Estern der Acryl- oder Methacrylsäure mit hydroxylgruppenhaltigen Polyestern oder Polyethern, wobei man zunächst einen Rohester herstellt, der noch Restmengen an Acryl- und oder Methacrylsäure enthält und eine Säurezahl (SZ) von x aufweist, wobei x eine Säurezahl von 20 mg KOH/g oder höher bedeutet, und diesen Rohester mit einer mindestens eine Carbodiimidgruppe (-N=C=N- Gruppe) pro Molekül enthaltenden Verbindung (C) derart umsetzt, bis eine Säurezahl y von 10 mg KOH/g oder weniger erreicht ist, wobei folgende beiden Maßgaben gelten: (i) die Menge der eingesetzten Verbindung (C) ist mindestens so groß, dass sie rechnerisch ausreicht, um die angestrebte Säurezahl y zu erreichen; (ii) die Menge der eingesetzten Verbindung (C) ist höchstens 1,2 mal so groß wie diejenige Menge der Verbindung (C), die rechnerisch benötigt wird, um die Säurezahl x des Rohesters auf einen Wert von 10 mg KOH/g abzusenken.

## Beschreibung

### Gebiet der Erfindung

Die vorliegende Erfindung betrifft ein verbessertes Verfahren zur Herstellung von Estern der Acryl- oder Methacrylsäure mit hydroxylgruppenhaltigen Polyestern oder Polyethern und deren Verwendung zur Herstellung strahlungshärtbarer Überzugsmassen, z.B. in Lack-Formulierungen, welche durch UV- bzw. Elektronenstrahlen schnell gehärtet werden können.

EP-B-126,341 beschreibt ein Verfahren zur Herstellung von Estern der (Meth)acrylsäure mit hydroxylgruppenhaltigen organischen Verbindungen, wobei man gesättigte, gegebenenfalls Ethergruppen enthaltende Polyester, die mindestens 2 freie Hydroxylgruppen (OH-Gruppen) pro Molekül enthalten, oder Polyether, die mindestens 2 freie Hydroxylgruppen (OH-Gruppen) pro Molekül enthalten, wobei diese Polyester oder Polyether durchschnittliche Molekulargewichte Mn zwischen 400 und 4000 g/mol aufweisen, mit 100 bis 150 Mol-% - bezogen auf die OH-Gruppen des Polyesters bzw. Polyethers - Acrylsäure oder Methacrylsäure in Gegenwart eines sauren Veresterungskatalysators und mindestens eines Kohlenwasserstoffs, der mit Wasser ein azeotropes Gemisch bildet, sowie geringer Mengen eines Polymerisationsinhibitors unter azeotroper Entfernung des bei der Veresterung entstehenden Wassers bei erhöhter Temperatur verestert, nach der Veresterung den Kohlenwasserstoff destillativ entfernt und nach Neutralisation des Veresterungskatalysators die restliche Acryl- oder Methacrylsäure mit einer der Säurezahl äquivalenten Menge einer mindestens zwei Epoxidgruppen pro Molekül aufweisenden Epoxidverbindung bis zu einer Säurezahl von 5 mg KOH/g oder weniger umsetzt.

Die EP-A-0,686,621 beschreibt die Verwendung quartärer Ammonium- oder PhosphoniumVerbindungen zur Beschleunigung der Umsetzung der Epoxidverbindungen.

Carbodiimide sind Verbindungen, die ein oder mehrere -N=C=N- Gruppen enthalten. Die Chemie der Carbodiimide ist beispielsweise im Review "Carbodiimide Chemistry: Recent Advances" beschrieben (Andrew Williams and Ibrahim T. Ibrahim, Chem. Rev. 1981, 81, 589 - 636).

### Beschreibung der Erfindung

Aufgabe der vorliegenden Erfindung war es, ein Verfahren zur Herstellung von Estern der (Meth)acrylsäure mit hydroxylgruppenhaltigen Polyestern oder Polyethern bereitzustellen, das den abschließenden Schritt des Verfahrens gemäß der EP-B-126, also die Absenkung der Säurezahl, dahingehend verändert, dass zur Erreichung der Säurezahl von 5 mg KOH/g oder weniger kürzere Reaktionszeiten und/oder geringere Temperaturen benötigt werden.

Gegenstand der vorliegenden Erfindung ist ein Verfahren zur Herstellung von Estern der (Meth)acrylsäure mit hydroxylgruppenhaltigen organischen Verbindungen, wobei man in einem ersten Schritt ein oder mehrere gesättigte, gegebenenfalls Ethergruppen enthaltende Polyester (P1), die mindestens 2 freie Hydroxylgruppen pro Molekül enthalten, und/oder ein oder mehrere Polyether (P2), die mindestens 2 freie Hydroxylgruppen (OH-Gruppen) pro Molekül enthalten, wobei die Verbindungen (P1) und (P2) durchschnittliche Molekulargewichte Mn zwischen 250 und 4000 aufweisen, mit 100 bis 150 Mol-% - bezogen auf die Gesamtmenge der freien OH-Gruppen in den Verbindungen (P1) und/oder (P2) - Acrylsäure und/oder Methacrylsäure in Gegenwart eines sauren Veresterungskatalysators und mindestens eines Kohlenwasserstoffs, der mit Wasser ein azeotropes Gemisch bildet, sowie geringer Mengen eines Polymerisationsinhibitors unter azeotroper Entfernung des bei der Veresterung entstehenden Wassers bei erhöhter Temperatur verestert, nach der Veresterung in einem zweiten Schritt den Kohlenwasserstoff destillativ entfernt und den nunmehr vorliegenden Rohester, der auf Grund der in ihm enthaltenen Restmengen an Acryl- und oder Methacrylsäure eine Säurezahl (SZ) von x mg KOH/g aufweist, wobei x eine Säurezahl von 20 mg KOH/g oder höher bedeutet, in einem dritten Schritt mit einer mindestens eine Carbodiimidgruppe ( -N=C=N- Gruppe ) pro Molekül enthaltenden Verbindung (C) derart umsetzt, bis eine Säurezahl y von 10 mg KOH/g oder weniger erreicht ist, wobei für den dritten Schritt folgende beiden Maßgaben gelten: (i) die Menge der eingesetzten Verbindung (C) ist mindestens so groß, dass sie rechnerisch ausreicht, um die angestrebte Säurezahl y zu erreichen; (ii) die Menge der eingesetzten Verbindung (C) ist höchstens 1,2 mal so groß wie diejenige Menge der Verbindung (C), die rechnerisch benötigt wird, um die Säurezahl x des Rohesters auf einen Wert von 10 mg KOH/g abzusenken.

Das erfindungsgemäße Verfahren umfasst mithin folgende drei Schritte:
1. Die Umsetzung der genannten OH-Gruppen-haltigen Polyether (P1) und/oder OH-Gruppen-haltiger Polyester (P2) mit (Meth)acrylsäure in einem Kohlenwasserstoff, der mit Wasser ein Azeotrop bilden kann, in Gegenwart eines sauren Veresterungskatalysators und eines Polymerisationsinhibitors unter azeotroper Entfernung des gebildeten Reaktionswassers.
2. Die Entfernung des Kohlenwasserstoffs.
3. Die Umsetzung von überschüssiger (Meth)acrylsäure mit einer mindestens eine Carbodiimidgruppe pro Molekül enthaltenden Verbindung (C), wobei die oben genannten Maßgaben gelten.

### Zu den Verbindungen (P1) und (P2)

In dem erfindungsgemäßen Verfahren werden im ersten Schritt zunächst die Hydroxylgruppen enthaltenden Verbindungen (P1) und/oder (P2) mit (Meth)acrylsäure verestert.

Bei den Verbindungen (P1) handelt es sich um gesättigte, gegebenenfalls Ethergruppen enthaltende Polyester, die mindestens 2 freie Hydroxylgruppen pro Molekül enthalten. Die Verbindungen (P1) weisen durchschnittliche Molekulargewichte Mn zwischen 250 und 4000 g/mol und insbesondere zwischen 400 und 2000 g/mol auf. Bei den Angaben für Mn handelt es sich um das zahlenmittlere Molekulargewicht. Wie oben ausgeführt können im ersten Schritt auch Mischungen unterschiedlicher Verbindungen (P1) eingesetzt werden.

Bei den Verbindungen (P2) handelt es sich um gesättigte, gegebenenfalls Ethergruppen enthaltende Polyether, die mindestens 2 freie Hydroxylgruppen pro Molekül enthalten. Die Verbindungen (P2) weisen durchschnittliche Molekulargewichte Mn zwischen 250 und 4000 g/mol und insbesondere zwischen 400 und 2000 g/mol auf. Bei den Angaben für Mn handelt es sich um das zahlenmittlere Molekulargewicht. Wie oben ausgeführt können im ersten Schritt auch Mischungen unterschiedlicher Verbindungen (P2) eingesetzt werden.

Die Verbindungen (P1) können beispielsweise durch Veresterung von Dicarbonsäuren mit Diolen und Triolen hergestellt werden. Die Umsetzung kann dabei in Substanz oder in Gegenwart eines Schleppmittels erfolgen. Vorzugsweise werden als Dicarbonsäuren Bernsteinsäure, Glutarsäure, Adipinsäure, Sebacinsäure, o-Phthalsäure, deren Isomere und Hydrierungsprodukte sowie veresterbare Derivate, wie Anhydride oder Dialkylester der genannten Säuren eingesetzt. Als Diole werden vorzugsweise Ethylenglykol, 1,2-Propylenglykol, 1,3-Propylenglykol, 1,4-Butandiol, 1,6-Hexandiol, Neopentylglykol, Cyclohexandimethanol sowie Polyglykole vom Typ des Polyethylenglykols und Polypropylenglykols eingesetzt. Als Triole sind Trimethylolpropan und Glycerin bevorzugt.

Zu den erfindungsgemäß einzusetzenden Verbindungen (P1) zählen auch Polycaprolactondiole und -triole, deren Herstellung dem Fachmann ebenfalls bekannt ist.

Als Verbindungen (P2) kommen beispielsweise solche in Frage, welche nach bekannten Verfahren durch Umsetzung von zwei- und/oder mehrwertigen Alkoholen mit verschiedenen Mengen an Ethylenoxid und/ oder Propylenoxid erhalten werden können. Bei den Ethylenglykol/Propylenglykol-Mischkondensationsprodukten wird die Umsetzung vorzugsweise so gesteuert, dass endständig überwiegend primäre Hydroxylgruppen entstehen. Desgleichen können auch Polymerisationsprodukte des Tetrahydrofurans oder Butylenoxids eingesetzt werden.

In einer Ausführungsform werden die Verbindungen (P1) und/oder (P2) in Schritt 1 als solche eingesetzt.

In einer weiteren Ausführungsform werden die Verbindungen (P1) und/oder (P2) in Schritt 1 in situ hergestellt.

### Zum Schritt 1 des Verfahrens

Die Verbindungen (P1) und/oder (P2) werden mit 100 bis 150 Mol.-% - bezogen auf die Gesamtmenge der freien OH-Gruppen in den Verbindungen (P1) und/oder (P2) - Acrylsäure und/oder Methacrylsäure versetzt und in Gegenwart eines sauren Veresterungskatalysators, sowie in Gegenwart eines Kohlenwasserstoffs, der mit Wasser ein azeotropes Gemisch bildet, vorzugsweise bis zu einem Umsatz von mindestens 85% - und insbesondere 90 bis 95% - der Gesamtheit der Hydroxylgruppen der Verbindungen (P1) und/oder (P2) verestert.

Als Veresterungskatalysatoren setzt man vorzugsweise Schwefelsäure oder p-Toluolsulfonsäure ein. Die Reaktionstemperatur der Veresterung wird vorzugsweise auf einen Wert im Bereich von 60 bis 140 °C eingestellt.

Das gebildete Reaktionswasser wird azeotrop entfernt. Als Schleppmittel werden Kohlenwasserstoffe eingesetzt, die mit Wasser ein Azeotrop bilden können. Dabei können aliphatische und aromatische Kohlenwasserstoffe eingesetzt werden. Beispiele hierfür sind: Alkane und Cycloalkane, wie n-Hexan, n-Heptan, n-Octan, Isooctan, Cyclohexan und Methylcyclohexan, Aromaten wie Benzol, Toluol und die Xylol-Isomeren, ferner sogenannte Spezialbenzine, welche Siedegrenzen zwischen 70 und 140 °C aufweisen.

Zur Vermeidung einer vorzeitigen Polymerisation wird die Veresterung in Gegenwart geringer Mengen eines Polymerisationsinhibitors durchgeführt. Die chemische Natur des Polymerisationsinhibitors unterliegt an sich keinen besonderen Einschränkungen. Vorzugsweise setzt man die dem Fachmann zur Verhinderung einer thermischen Polymerisation einschlägig bekannten Verbindungen ein, beispielsweise Hydrochinon, Hydrochinonmonoalkylether, 2,6-Di-t-butylphenols, N-Nitrosoamine, Phenothiazine oder Phosphorigsäureester. Sie werden vorzugsweise in Mengen von 0,001 bis 2,0 Gew.-% und insbesondere in Mengen von 0,005 bis 0,5 Gew.-% - bezogen auf die Summe der Verbindungen (P1), (P2) und (Meth)acrylsäure - eingesetzt.

### Zu Schritt 2 des Verfahrens

Im Anschluss an Schritt 1 wird der eingesetzte Kohlenwasserstoff aus dem Reaktionsgemisch destillativ, gegebenenfalls unter vermindertem Druck, entfernt. Am Ende von Schritt 2 liegt ein Rohester vor, der auf Grund der in ihm enthaltenen Restmengen an Acryl- und oder Methacrylsäure eine Säurezahl (SZ) von x mg KOH/g aufweist. Diese Säurezahl x wird im nachfolgend beschrieben dritten Schritt des erfindungsgemäßen Verfahrens auf einen Wert y von 10 mg KOH/g oder weniger und insbesondere 6 mg KOH/g oder weniger reduziert.

### Zu Schritt 3 des Verfahrens

Im dritten Schritt des erfindungsgemäßen Verfahrens wird der am Ende von Schritt 2 vorliegende Rohester, der auf Grund der in ihm enthaltenen Restmengen an Acryl- und oder Methacrylsäure eine Säurezahl (SZ) von x mg KOH/g aufweist - wobei x eine Säurezahl von 20 mg KOH/g oder höher bedeutet - mit einer mindestens eine Carbodiimidgruppe ( -N=C=N-Gruppe ) pro Molekül enthaltenden Verbindung (C) derart umsetzt, bis eine Säurezahl y von 10 mg KOH/g oder weniger erreicht ist, wobei für den dritten Schritt folgende beiden Maßgaben gelten:
(i) die Menge der eingesetzten Verbindung (C) ist mindestens so groß, dass sie rechnerisch ausreicht, um die angestrebte Säurezahl y zu erreichen;
(ii) die Menge der eingesetzten Verbindung (C) ist höchstens 1,2 mal so groß wie diejenige Menge der Verbindung (C), die rechnerisch benötigt wird, um die Säurezahl x des Rohesters auf einen Wert von 10 mg KOH/g abzusenken.

Vorzugsweise wird die Umsetzung in Schritt 3 so weit geführt, bis eine Säurezahl y von 6 mg KOH/g oder weniger erreicht ist.

In einer Ausführungsform ist die Menge der eingesetzten Verbindung (C) diejenige, die rechnerisch benötigt wird, um die Säurezahl x des Rohesters auf einen Wert von 0 mg KOH/g abzusenken. Dies bedeutet, dass man eine der Säurezahl x des Rohesters äquivalente Menge an Verbindung (C) einsetzt.

Die Verbindungen (C) werden nachfolgend abkürzend auch Carbodiimide genannt. Die Umsetzung der restlichen (Meth)acrylsäure mit dem Carbodiimid (C) erfolgt vorzugsweise bei 90 bis 130 °C und insbesondere bei 100 bis 110 °C. Es können ein oder mehrere Carbodiimide (C) eingesetzt werden.

Wie bereits gesagt müssen die Verbindungen (C) mindestens eine Carbodiimidgruppe pro Molekül enthaltenden.

Eine geeignete Gruppe von Verbindungen (C) sind dementsprechend die Monocarbodiimide, die genau eine Carbodiimidgruppe pro Molekül aufweisen. Beispiele für geeignete Monocarbodiimide sind Dicyclohexylcarbodiimid (DCC), Diisopropylcarbodiimid, 1-Ethyl-3-(3-dimethylaminopropyl)carbodiimid (EDC), Bis-ortho-tolylcarbodiimid, Bis-2,4,6-Triisopropylphenylcarbodiimid und Bis-2,6-Diisopropylphenylcarbodiimid.

Eine andere geeignete Gruppe von Verbindungen (C) sind die Polycarbodiimide, die zwei oder mehrere Carbodiimidgruppen pro Molekül aufweisen. Beispiele für geeignete Polycarbodiimide sind solche, die durch katalytische Kondensation aus Isophorondiisocyanat, 4,4'-Di(isocyanatocyclohexyl)methan, α, α, α', α'-Tetramethylxylylendiisocyanat, den Isomeren des Diisocyanatophenylmethans und 2,6-Diisopropylphenyldüsocyanat zugänglich sind.

In einer Ausführungsform wird Schritt in Gegenwart eines Katalysators durchgeführt, der die Umsetzung von Acryl- und/oder Methacrylsäure mit dem Carbodiimid (C) zu katalysieren vermag. Beispiele für geeignete Katalysatoren sind etwa Triphenylphosphin, Tributylamin, Tetrabutylammmoniumbromid sowie Dimethylethanolamin. Vorzugsweise werden diese Katalysatoren in Mengen im Bereich von 0,5 bis 2,5 Gew.-% - bezogen auf die gesamten Reaktionsmischung - eingesetzt.

### Verwendung

Ein weiterer Erfindungsgegenstand ist die Verwendung der nach dem erfindungsgemäßen Verfahren zugänglichen Polyol(meth)acrylate für strahlungshärtbare Überzugsmassen. Dabei können die nach dem erfindungsgemäßen Verfahren zugänglichen Polyol(meth)acrylate als solche eingesetzt werden oder in Abmischung mit weiteren strahlungshärtbaren Acrylesterverbindungen. Beispielhaft seien genannt: 4-t-Butylcyclohexylacrylat, Phenoxyethylacrylat, Hexandioldiacrylat, Tripropylenglykoldiacrylat und Trimethylolpropantriacrylat.

Die Härtung kann durch Elektronenstrahlen oder durch UV-Strahlung bewirkt werden. Bei der UV-Härtung werden vorzugsweise Photoinitiatoren zugesetzt.

### Beispiele

### Eingesetzte Carbodiimide

CD1: Bis-2,6-diisopropylphenylcarbodiimid. Dieses Carbodiimid wurde in den Beispielen 1 und 3 eingesetzt.

CD2: Carbodiimid, hergestellt gemäß Beispiel 2 des europäischen Patents EP 628,541 B1. Dieses Carbodiimid wies einen NCN-Gehalt von 8 Gew.% - bezogen auf das Carbodiimid - auf und wurde in den Beispielen 2, 4, 5 und 6 eingesetzt.

### Herstellung des Rohesters

In einer 2-Liter Apparatur eine Mischung von 694,6 g ethoxyliertes Trimethylolpropan (mit einer OH-Zahl von 607 mg KOH/g), 190 g Adipinsäure, 327,3 g Methylcyclohexan, 424,5 g Acrylsäure, 1,3g Hypophosphorige Säure und 6,5 g conc. Schwefelsäure vorgelegt. Zu dieser Mischung wurden als Stabilisatoren 1,3g 2,6 tert-butyl-p-Kresol (=Kerobit ), 3,9 g Methylhydrochinon und 0,04g Phenothiazin zudosiert. Das Gesamtsystem wurde auf eine Temperatur von 109 °C erhitzt. Innerhalb von 6,5 Stunden wurden 156 g Wasser ausgekreist.

Anschließend wurden das Methylcyclohexan und überschüssige Acrylsäure bis zu einer Säurezahl (SZ) von 42 mg KOH/g Substanz im Vakuum entfernt.

Der so erhaltene Rohester mit der SZ von 42 mg KOH/g wurde in den nachfolgend beschriebenen Beispielen (erfindungsgemäße Beispiele 1 bis 6) und Vergleichsbeispielen eingesetzt.

### Beispiel 1

Zu 170 g des Rohesters wurden bei 60 °C 65,43 g Bis-2,6-diisopropylphenylcarbodiimid (CD1) gegeben. Die Mischung wurde auf 108 °C aufgeheizt und zwei Stunden gerührt. Nach dieser Zeit betrug die SZ 4,81 mg KOH/g.

### Beispiel 2

Zu 254,28 g des Rohesters wurden bei 108 °C 95,25 g des Carbodiimids CD2 gegeben. Die Mischung wurde eine Stunde bei 108°C gerührt. Nach dieser Zeit betrug die SZ 3,9 mg KOH/g.

### Beispiel 3

Zu 170 g des Rohesters wurden bei 40 °C 65,43 g Bis-2,6-diisopropylphenylcarbodiimid (CD1) gegeben. Die Mischung wurde auf 60 °C aufgeheizt und drei Stunden gerührt. Nach dieser Zeit betrug die SZ 9,0 mg KOH/g. Danach wurde die Mischung auf 108 °C geheizt und für weitere vier Stunden gerührt. Nach dieser Zeit betrug die SZ 4,6 mg KOH/g.

### Beispiel 4

Zu 170 g des Rohesters wurden bei 30 °C 77,43 g des Carbodiimids CD2 gegeben. Die Mischung wurde viereinhalb Stunden bei 60 °C gerührt. Nach dieser Zeit betrug die SZ 5 mg/KOH g.

### Beispiel 5

Zu 170 g des Rohesters wurden bei 20 °C 94,77 g des Carbodiimids CD2 gegeben. Die Mischung wurde bei 30 °C für siebeneinhalb Stunden gerührt. Nach dieser Zeit betrug die SZ 5,70 mg/KOH g.

### Beispiel 6

Zu 170 g des Rohesters wurden bei 30 °C 98,52 g des Carbodiimids CD2 gegeben. Die Mischung wurde sechs Stunden bei 40 °C gerührt. Nach dieser Zeit betrug die SZ 6,7 mg/KOH g.Danach wurde die Mischung auf 108 °C geheizt und für weitere zwei Stunden gerührt. Nach dieser Zeit betrug die SZ 4,86 mg KOH/g.

### Vergleichsbeispiel 1

Zu 250g Rohester wurden 4,28g des Katalysators Triphenylphosphin und 35,43 g Bisphenol-A-diglycidether bei einer Temperatur von 106 bis 108 °C zugegeben. Nach 6,4 Stunden Reaktionsdauer bei 108 °C betrug die SZ 4,8 mg KOH/g.

### Vergleichsbeispiel 2

Zu 500g Rohester wurden 10,5g des Katalysators Tributylamin und 70,6 g Bisphenol-A-diglycidether bei einer Temperatur von 108 °C zugegeben. Nach 7 Stunden Reaktionsdauer bei 108 °C betrug die SZ 3,9 mg KOH/g.

## Patentansprüche

1. Verfahren zur Herstellung von Estern der (Meth)acrylsäure mit hydroxylgruppenhaltigen organischen Verbindungen, wobei man in einem ersten Schritt ein oder mehrere gesättigte, gegebenenfalls Ethergruppen enthaltende Polyester (P1), die mindestens 2 freie Hydroxylgruppen pro Molekül enthalten, und/oder ein oder mehrere Polyether (P2), die mindestens 2 freie Hydroxylgruppen (OH-Gruppen) pro Molekül enthalten, wobei die Verbindungen (P1) und (P2) durchschnittliche Molekulargewichte Mn zwischen 250 und 4000 aufweisen, mit 100 bis 150 Mol-% - bezogen auf die Gesamtmenge der freien OH-Gruppen in den Verbindungen (P1) und/oder (P2) - Acrylsäure und/oder Methacrylsäure in Gegenwart eines sauren Veresterungskatalysators und mindestens eines Kohlenwasserstoffs, der mit Wasser ein azeotropes Gemisch bildet, sowie geringer Mengen eines Polymerisationsinhibitors unter azeotroper Entfernung des bei der Veresterung entstehenden Wassers bei erhöhter Temperatur verestert, nach der Veresterung in einem zweiten Schritt den Kohlenwasserstoff destillativ entfernt und den nunmehr vorliegenden Rohester, der auf Grund der in ihm enthaltenen Restmengen an Acryl- und oder Methacrylsäure eine Säurezahl (SZ) von x aufweist, wobei x eine Säurezahl von 20 mg KOH/g oder höher bedeutet, in einem dritten Schritt mit einer mindestens eine Carbodiimidgruppe ( -N=C=N- Gruppe ) pro Molekül enthaltenden Verbindung (C) derart umsetzt, bis eine Säurezahl y von 10 mg KOH/g oder weniger erreicht ist, wobei für den dritten Schritt folgende beiden Maßgaben gelten: (i) die Menge der eingesetzten Verbindung (C) ist mindestens so groß, dass sie rechnerisch ausreicht, um die angestrebte Säurezahl y zu erreichen; (ii) die Menge der eingesetzten Verbindung (C) ist höchstens 1,2 mal so groß wie diejenige Menge der Verbindung (C), die rechnerisch benötigt wird, um die Säurezahl x des Rohesters auf einen Wert von 10 mg KOH/g abzusenken.

2. Verwendung der nach dem Verfahren gemäß Anspruch 1 zugänglichen Polyol(meth)acrylate für strahlungshärtbare Überzugsmassen.
